# EUROPEAN PATENT APPLICATION

(11) **EP 3 111 909 A1**
(43) Date of publication of application: **04.01.2017**
(21) Application number: 15754593.0
(22) Date of filing: 06.02.2015
(51) Int. Cl.: A61H 15/00

(54) **COSMETIC DEVICE**

(30) Priority: 28.02.2014 JP 2014039965
(71) Applicant: MTG Co., Ltd., Aichi 453-0041 (JP)
(72) Inventor: MATSUSHITA Tsuyoshi, Nagoya-shi Aichi 453-0041 (JP)
(74) Representative: Kramer Barske Schmidtchen Patentanwälte PartG mbB
(86) International application number: PCT/JP2015/053421
(87) International publication number: WO 2015/129435

(57) **Abstract**

A cosmetic device 1 includes a main body 10, at least four support shafts 40a to 40d, rollers 20a to 20d, and a handle 11. The support shafts 40a to 40d are supported by the main body 10, extending from the main body 10 so as to go away from each other. The rollers 20a to 20d are supported by the support shafts 40a to 40d so as to be rotatable about axis lines La to Ld of the support shaft 40a to 40d, respectively.

## Description

### TECHNICAL FIELD

The present invention relates to a cosmetic device.

### BACKGROUND ART

There have been proposed various kinds of cosmetic devices that exert a massaging effect by, for example, pressing skin with rollers. Examples of such cosmetic devices include Non Patent Document 1, which discloses a cosmetic device including a main body with four rollers. The main body has, in plan view from a top surface side, a rectangular shape in which the both long sides of the rectangular shape are gradually dished inwardly, and the both short sides of the rectangular shape are gradually swelled outwardly. In addition, in the central portion of the main body, in plan view, a top surface gradually swells, and on a bottom surface side, rollers are arranged at four corner portions, respectively. The axis lines of the rollers extend so as to go away from each other. In the cosmetic device, the main body is directly held with fingers put along both the long sides of the main body. Then, by being moved in a long side direction or a short side direction with the rollers brought into contact with a skin surface, the cosmetic device presses the skin with two leading rollers and picks up the skin with two following rollers, exerting the massaging effect.

### PRIOR ART DOCUMENT

### Non Patent Document

Non Patent Document 1: "ReFa for Body", [online], MTG Co., Ltd., [Searched on 18 Dec. 2013], Internet URL: http://www.mtg.gr.jp/products/beauty/product/refa_for-body/ index.html

### SUMMARY OF THE INVENTION

### PROBLEM TO BE SOLVED BY THE INVENTION

However, in the cosmetic device disclosed in Non Patent Document 1, the main body in which the rollers are arranged is directly held in use. Thus, when the cosmetic device is used in a portion like a face that curves in various directions, it is necessary to greatly change the direction of a hand holding the main body or greatly twist a wrist, along the curves of the skin in order to move the cosmetic device while running the rollers along the skin of the portion. For this reason, the cosmetic device is less user-friendly, and the massaging effect may not be exerted sufficiently because it is difficult to move the rollers while keeping a proper angle with respect to skin.

The present invention, which has been made in such circumstances, provides a cosmetic device that is suitable to massage portions, such as those of a face curving in various directions.

### MEANS FOR SOLVING THE PROBLEM

An aspect of the present invention is a cosmetic device including:
a main body;
four support shafts that are supported by the main body and extend from the main body so as to go away from each other;
rollers that are supported by the support shafts so as to be rotatable about axis lines of the support shafts; and
a handle that extends from the main body.

### EFFECTS OF THE INVENTION

In the cosmetic device, the main body is provided with the handle. This configuration allows the cosmetic device to be used with the handle held, eliminating the necessity of excessively greatly changing the direction of a hand holding the handle or the necessity of excessively greatly twisting a wrist when moving the rollers along skin of a portion like a face that curves in various directions. As a result, the user-friendliness of the cosmetic device is improved as compared with the case where the main body is directly held, and it is possible to facilitate the movement of the rollers while keeping a proper angle with respect to a portion like a face that curves in various directions, providing a sufficient massaging effect.

As seen from the above, according to the present invention, it is possible to provide a cosmetic device that is suitable to massage a portion like a face that curves in various directions.

### BRIEF DESCRIPTION OF THE DRAWINGS

[Figure 1] Figure 1 is a front view of a cosmetic device in Embodiment 1.
[Figure 2] Figure 2 is a right side view of the cosmetic device in Embodiment 1.
[Figure 3] Figure 3 is a rear view of the cosmetic device in Embodiment 1.
[Figure 4] Figure 4 is a cross sectional view taken at a position along a line IV-IV in Figure 1.
[Figure 5] Figure 5 is a cross sectional view taken at a position along a line V-V in Figure 2.
[Figure 6] Figure 6 is a cross sectional view taken at a position along a line VI-VI in Figure 2.
[Figure 7] Figure 7 is a top view illustrating a usage of the cosmetic device in Embodiment 1.
[Figure 8] Figure 8 is a bottom view illustrating a usage of the cosmetic device in Embodiment 1.
[Figure 9] Figure 9 is a side view illustrating a usage of the cosmetic device in Embodiment 1.
[Figure 10] Figure 10 is a front view illustrating a usage of the cosmetic device in Embodiment 1.

### MODE FOR CARRYING OUT THE INVENTION

The handle is connected to the main body via a connecting portion, and the connecting portion can be narrowed. In this case, the connecting portion between the main body and the handle is narrowed and thus guides fingers along the narrowed portion of the connecting portion when the handle is held, making the handle easy to be held. Thus, when the cosmetic device is used for a portion like a face that curves in various directions, it is possible to hold the handle sufficiently even when the direction of a hand holding the handle is changed, whereby the usability of the cosmetic device is enhanced.

For the cosmetic device, the expression "the connecting portion between the main body and the handle is narrowed" means that the connecting portion is formed so as to become thin first and then become thick as extending from a main body side toward a handle side. In other words, the expression means that, considering a cross section of the connecting portion perpendicular to a direction from the main body side toward the handle side, as the position of the cross section shifts from the main body side toward the handle side, the area of the external shape of the cross section first becomes small as shifting from the main body side toward the handle side, and then becomes large as shifting further toward the handle side.

It is preferable that the rollers include the first pair of rollers adjacent to each other and the second pair of rollers adjacent to each other, the first pair of rollers and the second pair of rollers are aligned along the direction in which the handle extends, and when viewed in the direction of arranging the first pair of rollers, the connecting portion includes a surface that is on a side opposite to a side toward which the support shafts extend so as to go away from each other and is formed so as to have a gentle curve, and a surface that is on the side toward which the support shafts extend so as to go away from each other and is formed so as to be recessed and narrowed. The configuration of this case facilitates guiding the rollers to a position on a palm side when the handle is held, and thus facilitates keeping a proper angle of the rollers with respect to skin. The user-friendliness of the cosmetic device 1 is thereby improved.

It is preferable that the rollers include the first pair of rollers that are adjacent to each other, and the second pair of rollers that are adjacent to each other, and when viewed in a direction arranging the first pair of rollers, the first inclination angle of axis lines of the first pair of rollers with respect to an imaginary perpendicular is smaller than the second inclination angle of axis lines of the second pair of rollers with respect to the imaginary perpendicular, the imaginary perpendicular being perpendicular to an imaginary plane in contact with the rollers. In this case, when the cosmetic device is used in reciprocating operation in a direction from the second pair of rollers toward the first pair of rollers, with the first pair of rollers and the second pair of rollers made to abut a skin surface, an effect of moderately pressing the skin surface by the leading pair of rollers can be exerted, and an effect of moderately picking up the skin by the following pair of rollers can be exerted, also on the skin surface of a curved portion.

Moreover, the cosmetic device can be used as follows. First, the cosmetic device is moved in a direction from the second pair of rollers toward the first pair of rollers, with the second pair of rollers separated from a skin surface, and with the first pair of rollers made to abut the skin surface, and then the second pair of rollers are made to abut the skin surface (a first stage). Subsequently, the cosmetic device is moved in the direction from the second pair of rollers toward the first pair of rollers, with the first pair of rollers and the second pair of rollers made to abut the skin surface (a second stage). Furthermore, while the cosmetic device is moved in the direction from the second pair of rollers toward the first pair of rollers, the first pair of rollers are made separated from the skin surface (a third stage).

Through the above-described usage of the cosmetic device, first, the first stage has an effect of moderately picking up the skin surface by the first pair of rollers abutting the skin surface. Moreover, the second stage has an effect of keeping the skin picked up by the leading first pair of rollers, and moderately picking up the skin surface by the following second pair of rollers. Subsequently, the third stage has an effect of releasing the picking up of the skin by the first pair of rollers, and continuing the moderately picking up of the skin by the following second pair of rollers. In addition, in the case where, when viewed in the direction of arranging the first pair of rollers, the first inclination angle of the axis lines of the first pair of rollers with respect to the imaginary perpendicular is smaller than the second inclination angle of the axis lines of the second pair of rollers with respect to the imaginary perpendicular, the imaginary perpendicular being perpendicular to the imaginary plane in contact with the rollers, the amount of picking up the skin by the first pair of rollers is larger than the amount of picking up by the second pair of rollers. Therefore, the picking-up effect by the first pair of rollers in the first stage and the second stage is exerted relatively weakly, and the picking-up effect by the second pair of rollers in the second stage and the third stage is exerted relatively strongly. This configuration imparts differences in the skin-picking-up effect, further enhancing the massaging effect. In addition, exerting the picking-up effect in two strengths enables a user to recognize more clearly that skin is picked up, whereby the usability of the cosmetic device 1 can be enhanced.

It is preferable that the handle extends in a direction from the first pair of rollers toward the second pair of rollers. The configuration of this case facilitates a reciprocating operation of the cosmetic device in the direction from the first pair of rollers toward the second pair of rollers, with the handle held. As a result, it is possible to exert easily, of the first pair of rollers and the second pair of rollers, the skin-pressing effect by the leading pair of rollers and the picking-up effect by the following pair of rollers, whereby the operability of the cosmetic device is enhanced.

It is preferable that, when viewed from a side toward which the support shafts extend, the main body is formed so as to be wider than the connecting portion. In this case, the rigidity of the main body can be increased. In the cosmetic device, the rollers attached to the support shafts provided in the main body so as to go away from each other are made to abut skin in the state where the handle that extends from the main body is held. Thus, in use, the main body receives, via the support shafts, loads in directions in which the rollers are separated from one another, and the connecting portion with the extending handle also receives a load. However, since the rigidity of the main body is increased as previously described, the main body is inhibited from yielding at these loads. It is thereby possible to cause the rollers to abut the skin surface reliably, whereby providing a high massaging effect even in use in a curved portion. In addition, the main body is excellent in durability, whereby the cosmetic device is made suitable to use for a portion that curves in various directions.

It is preferable that the support shafts are covered with the main body and the rollers and are not exposed. The configuration of this case prevents skin, hair, or the like from contacting with the support shafts in use. It is thus possible to prevent a user from bearing an unnecessary burden, whereby the usability of the cosmetic device is enhanced.

The diameter of the rollers can be set to 20 to 40 mm, preferably to 25 to 35 mm, more preferably to 28 to 32 mm. This configuration prevents the rollers from being excessively large or small, and a moderate skin-pressing effect and skin-picking-up effect are exerted on a portion like a face that curves in various directions. In addition, this configuration prevents the main body to which the rollers are arranged from being excessively large or small relative to the handle, and makes the size of the main body moderate. Therefore, the cosmetic device is well-balanced in weight, whereby the cosmetic device is made excellent in operability.

It is preferable that, when viewed in a direction from the handle to the main body, a supporting shaft mounting surface, which is a surface on a side on which the support shafts are provided in the main body, is formed so as to be wider than the handle, the support shafts are provided through the supporting shaft mounting surface at predetermined intervals, and the rollers are positioned on a side toward which the support shafts extend from the supporting shaft mounting surface. The configuration of this case enables the adjacent rollers to be disposed at the predetermined intervals while maintaining the rigidity of the main body. It is thereby possible to retain the adjacent rollers at the predetermined intervals even when the handle is held to press the rollers against the skin surface of a portion like a face that curves in various directions. This retention enables moderate skin-pressing effect and skin-picking-up effect to be exerted more effectively on a curved portion, whereby the cosmetic device is made suitable to use for a portion that curves in various directions.

It is preferable that the handle is substantially rigid. The configuration of this case makes the handle hard to yield, whereby force given to the handle can be exactly transmitted to the rollers. Therefore, by changing the strength of force given to the handle, it is possible to easily perform pressing and picking up with an appropriate strength for a portion to be massaged, exerting a high massaging effect. "Substantially rigid" refers to a body that has a rigidity to a degree at which the body hardly yields at force given to the handle in a usual usage of the cosmetic device.

It is preferable that the support shafts are rotatably supported by the main body, and the rollers are supported by the support shafts in such a manner that the rollers are rotatable integrally with the support shafts. In this case, the main body may be provided with a structure that makes the support shafts rotatable, eliminating the necessity of providing a structure inside the rollers to make the rollers rotatable, whereby the freedom in design of the rollers is enhanced.

### EMBODIMENT

### (Embodiment 1)

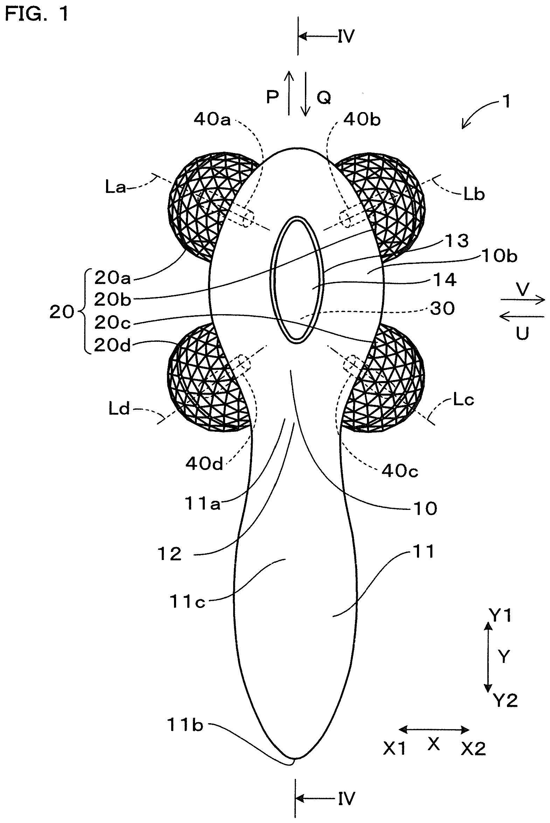

An embodiment of a cosmetic device will be described with reference to Figure 1 to Figure 10.

As illustrated in Figure 1, a cosmetic device 1 in the present embodiment includes a main body 10, four support shafts 40a to 40d, rollers 20, and a handle 11.

The support shafts 40a to 40d are supported by the main body 10, extending from the main body 10 so as to go away from each other.

As the rollers 20, four rollers are included: a first roller 20a, a second roller 20b, a third roller 20c, and a fourth roller 20d, which are supported by the support shafts 40a to 40d so as to be rotatable about axis lines La to Ld of the support shafts 40a to 40d, respectively.

The handle 11 extends from the main body 10.

In addition, a connecting portion 12 between the main body 10 and the handle 11 is narrowed.

There will be described below in detail the cosmetic device 1 in the present embodiment.

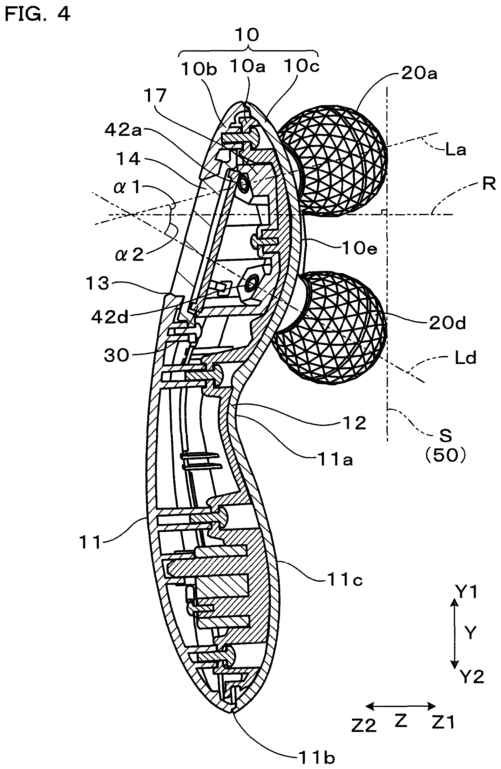

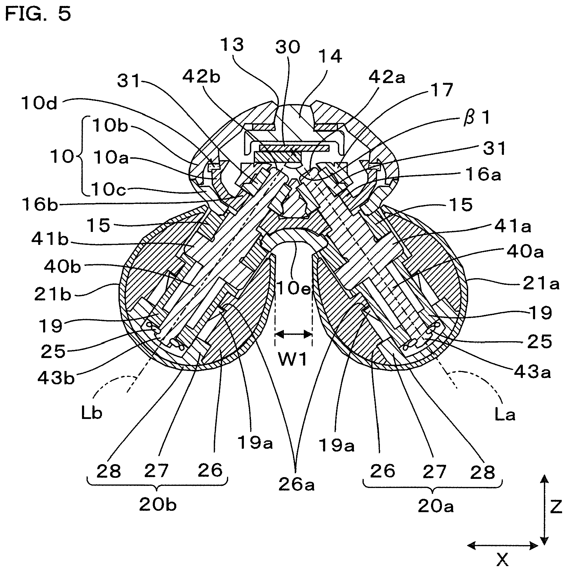

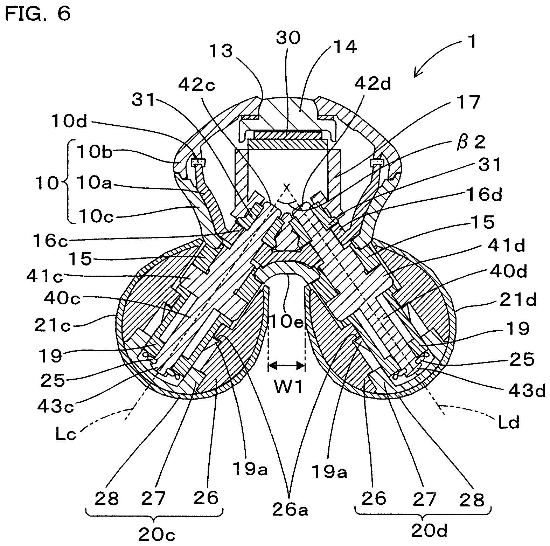

The main body 10 is made of an ABS resin and chromium-plated. As illustrated in Figure 4, the main body 10 includes a body base 10a, an upper body cover 10b, and a lower body cover 10c. To the body base 10a, a shaft holder 17 is screwed. The body base 10a is screwed to the upper body cover 10b. As illustrated in Figure 5 and Figure 6, a resin-made gasket 10d is inserted between the body base 10a and the upper body cover 10b, that is, in an abutment portion between the body base 10a and the upper body cover 10b. The gasket 10d seals the gap between the body base 10a and the upper body cover 10b. The main body 10 is provided therein with a photovoltaic panel 30, which will be described later. In addition, in the middle of the main body 10, a window portion 13 is provided, which will be described later.

Furthermore, as illustrated in Figure 4, on the opposite side of the upper body cover 10b across the body base 10a, the lower body cover 10c is provided. On the lower body cover 10c, engaging hooks (not illustrated) are formed, and the engaging hooks are fitted into engaging grooves (not illustrated) formed on the body base 10a, whereby the lower body cover 10c is fixed to the body base 10a.

It is noted that the longitudinal direction of the main body 10 is denoted by Y, a direction of stacking the body base 10a, the upper body cover portion 10b, and the lower body cover 10c (thickness direction) is denoted by Z, and a direction perpendicular to both the longitudinal direction Y and the thickness direction Z (width direction) is denoted by X.

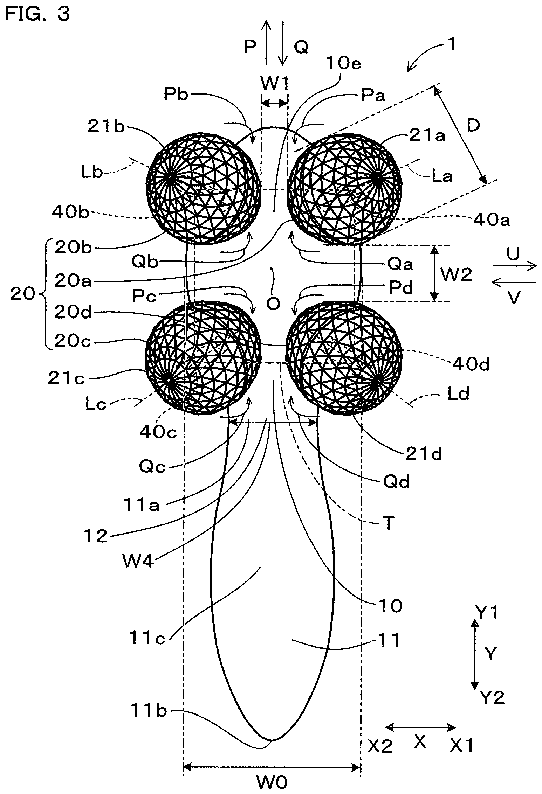

As illustrated in Figure 1, the main body 10 has an elliptical shape, in plan view, extending from a side in the first direction (Y1 direction) of the longitudinal direction Y to an opposite side in the second direction (Y2 direction) of the longitudinal direction Y. As illustrated in Figure 3, when viewed from a side toward which the support shafts 40a to 40d extend, the main body 10 is formed so as to be wider than a width W4 of the connecting portion 12, which will be described later. That is, a length W0 of the main body 10 in the width direction X is larger than the width W4 of the connecting portion 12. W0 can be set to 30 to 70 mm and is set to 46 mm in the present embodiment. W4 can be set to 15 to 30 mm and is set to 20 mm in the present embodiment.

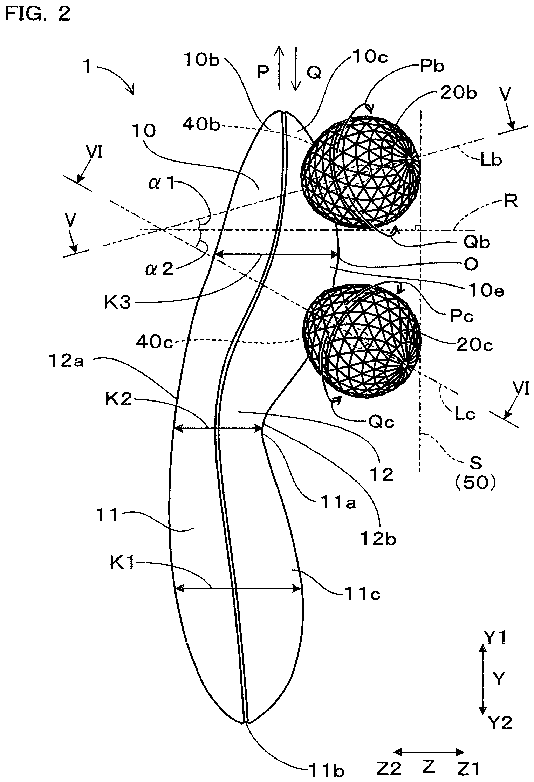

As illustrated in Figure 3, a surface on a lower body cover 10c side in the main body 10 (a side in the first direction Z1 of the thickness direction Z) serves as a supporting shaft mounting surface 10e through which the support shafts 40a to 40d are provided at predetermined intervals. The main body 10 bends convexly to project toward the side toward which the support shafts 40a to 40d extend, as illustrated in Figure 2. In addition, as illustrated in Figure 2, the main body 10 projects the most toward the side toward which the support shafts 40a to 40d extend, at a position O that is, as illustrated in Figure 3, the same as the center position of the four rollers 20a to 20b when viewed from the side toward which the support shafts 40a to 40d extend.

As illustrated in Figure 1, the handle 11 extends from the main body 10 in the Y2 direction. The main body 10 is formed integrally with the handle 11. In the present embodiment, the handle 11 is substantially rigid. "Substantially rigid" refers to a body that has a rigidity to a degree at which the body hardly yields at force given to the handle in a usual usage of the cosmetic device 1, and in the present embodiment, a formation material of the handle 11 is made of an ABS resin so as to be substantially rigid as with the main body 10. As the formation material to make the handle 11 substantially rigid, a wide variety of materials can be employed, including an ABS resin, as well as a reinforced plastic, a wood, a stone, a ceramic material, a stainless, and the like. The handle 11 has a substantially stick shape in plan view and swells, at a central portion 11c of the handle 11, toward a lower body cover 10c side (the side in the first direction Z1 of the thickness direction Z) as illustrated in Figure 2. In addition, the handle 11 radially contracts as extending toward a first end portion 11a and a second end portion 11b on the opposite side. The handle 11 is then connected to the main body 10 at the first end portion 11a, forming the connecting portion 12. In this way, the handle 11 inclines slightly toward the lower body cover 10c side (the side in the first direction Z1 of the thickness direction Z) with respect to the main body 10.

As illustrated in Figure 2, when viewed laterally, a thickness K1 of the thickest portion of the handle 11 can be set to 25 to 45 mm and is set to 30 mm in the present embodiment. In addition, a thickness K2 of the thinnest portion of the handle 11 (the first end portion 11a) can be set to 15 to 30 mm and is set to 20 mm in the present embodiment. In the main body 10, when viewed laterally, a thickness K3 of the thickest portion can be set to 25 to 45 mm and is set to 30 mm in the present embodiment. The connecting portion 12 includes the surface 12a on a side (Z2 side) opposite to the side toward which the above-described support shafts 40a to 40b extend so as to go away from each other, and a surface 12b on a side (Z1 side) toward which the support shafts 40a to 40b extend so as to go away from each other, and when viewed in a direction (the width direction X, see Figure 3) of arranging the first pair of rollers to be described later (the first roller 20a and the second roller 20b), the surface 12a is formed so as to have a gentle curve, and the surface 12b is formed so as to be recessed and narrowed. That is, the connecting portion 12 is formed so as to be narrowed. In the present embodiment, the connecting portion 12 is narrowed so as to be recessed toward a side (in the Z2 direction) opposite to the side toward which the support shafts 40a to 40d extend.

As illustrated in Figure 4 to Figure 6, four supporting barrels 16a to 16d are formed integrally with the body base 10a and the lower cover 10c. Through the supporting barrels 16a to 16d, the support shafts 40a, 40b, 40c, 40d are inserted, respectively. As illustrated in Figure 3, the support shafts 40a to 40d each have a substantially columnar shape and include, respectively, flange portions 41a to 41d each of which is formed projecting outward in a radial direction at a substantially central portion in an axis direction. The support shafts 40a to 40d are each made of a brass and chromium plated, and each have conductivity. The support shafts 40a to 40d extend from the body base 10a of the main body 10 toward the side in the first direction Z1 of the thickness direction Z so as to go away from each other. That is, as illustrated in Figure 3, when viewed from the lower body cover 10c side (the side in the first direction Z1 of the thickness direction Z), the support shafts 40a to 40d extend radially so as to come away from each other. As illustrated in Figure 5 and Figure 6, the support shafts 40a to 40d are covered with the main body 10 and the rollers 20 and are not exposed. Each of the support shafts 40a to 40d is not necessarily brass-made, and can be stainless-steel-made.

As illustrated in Figure 5 and Figure 6, the support shafts 40a to 40d include, on a main body 10 side, first end portions 42a, 42b, 42c, and 42d each formed with a thread groove to which a flange nut 31 is attached. The outer diameter of the flange nut 31 is larger than the inner diameter of the supporting barrels 16a to 16d of the body base 10a, and thus attaching the flange nuts 31 prevents the support shafts 40a to 40d from coming off from the supporting barrels 16a to 16d. On the inside of each flange nut 31, a ring-shaped nylon-resin member is provided, which inhibits the flange nut 31 from loosening.

The first end portions 42a, 42b, 42c, and 42d of the support shafts 40a to 40d are connected to the shaft holder 17 that is arranged inside the main body 10. The shaft holder 17 is made of an ABS resin. The shaft holder 17 is provided with a lead wire (not illustrated) that is electrically connected to the photovoltaic panel 30, which will be described later, and the lead wire electrically connects the photovoltaic panel 30 to the support shafts 40a to 40d. Instead of using the lead wire, the conductivity may be given by plating the ABS-resin-made shaft holder 17 with a metal, and the support shafts 40a to 40d and the photovoltaic panel 30 may be electrically connected via the shaft holder 17.

The support shafts 40a to 40d include, on the opposite side across the main body 10, second end portions 43a to 43d into which cylindrical bearing members 19 are fitted. The bearing members 19 are each made up of a synthetic-resin-made bearing having a cylindrical shape, and each include inner and outer circumferences that are plated with a chromium. The second end portions 43a to 43d of the support shafts 40a to 40d each project from the bearing member 19, and a C-ring 25 is inserted into the projection. Inserting the C-rings 25 prevents the support shafts 40a to 40d from coming off from the bearing members 19. In addition, on the outer circumference of each bearing member 19, a pair of locking hooks 19a are provided in a projecting manner, the locking hooks 19a being elastically deformable. Instead of plating the bearing members 19 with a chromium, the bearing members 19 may be formed of a conductive material.

Through the bearing members 19, the rollers 20a to 20d are inserted and rotatably supported. The shape of the rollers 20a to 20d is spherically formed. In the present specification, the term "spherical" is not necessarily assumed to be a strictly spherical shape, but is assumed to have a broad concept including not only a strict spherical shape but also shapes that can be recognized as a substantially spherical shape, such as an oval-sphere shape made by compressing a spherical shape, and a shape made by stretching a spherical shape in a predetermined direction. In addition, it is assumed that in the present specification that even a roller having a surface with minute grooves or protrusions formed thereon is considered to be "spherical" as long as the roller has a substantially spherical shape as a whole. In the present embodiment, the rollers 20a to 20d have a shape made by stretching a spherical shape toward the main body 10 side. In addition, the rollers 20a to 20d are hollow and each have an opening in a portion facing the main body 10. The rollers 20a to 20d each include an ABS-resin-made roller core 26, a PC-resin-made cap 27 that is fit on a front-end inner circumference of the roller core 26, and a synthetic-resin-made roller coating portion 28 that is formed covering the outer circumference the roller core 26.

Each roller coating portion 28 is plated with a platinum, which serves as a conductive portion, and is electrically connected to the chromium plate of the bearing member 19. On the inner circumference of each roller core 26, a step portion 26a is formed, which is engageable with the locking hook 19a of the bearing member 19. With the rollers 20a to 20d inserted into the bearing members 19, each locking hook 19a is engaged with the step portion 26a, and the rollers 20a to 20d are retained with respect to the bearing members 19. This configuration makes the rollers 20a to 20d attached rotatably, via the bearing members 19, about the axis lines La, Lb, Lc, and Ld of the support shafts 40a to 40d, respectively. In addition, as illustrated in Figure 3, outer circumferential surfaces 21a to 21d of the respective rollers 20a to 20d are subjected to what is called diamond cut, which is continuous formation of a large number of triangular planes.

As illustrated in Figure 5 and Figure 6, between the lower body cover 10c and the rollers 20a to 20d, insulating collars 15 are provided, respectively. The insulating collars 15 are each a resin-made annular member having insulation properties. The insulating collars 15 are inserted into the support shafts 40a to 40d, abut the flange portions 41a to 41d formed in the middles of the support shafts 40a to 40d, and also abut the lower body cover 10c. This configuration seals between the four supporting barrels 16a to 16d of the lower body cover 10c and the support shafts 40a to 40d. In addition, this configuration prevents the support shafts 40a to 40d from being unstable. Furthermore, this configuration secures electrical insulation properties between the conductive portion of the outer surface of the lower body cover 10c and the conductive portions of the outer circumferential surfaces 21a to 21d of the rollers 20a to 20d.

As illustrated in Figure 3, in the present embodiment, the four rollers 20a to 20d are arranged such that the centers of the rollers 20a to 20d are positioned at vertex positions of an imaginary rectangle T. The imaginary rectangle T has a rectangular shape that is long in the longitudinal direction Y. Therefore, of the four rollers 20a to 20d, the direction of arranging the first pair of rollers (the first roller 20a and the second roller 20b) is parallel to a direction of arranging the second pair of rollers (the third roller 20c and the fourth roller 20d). In addition, of the four rollers 20a to 20d, the direction of arranging the first pair of rollers (the first roller 20a and the second roller 20b) matches the width direction X, and the direction of arranging the second pair of rollers (the third roller 20c and the fourth roller 20d) also matches the width direction X. The first pair of rollers and the second pair of rollers are aligned along the direction in which the handle 11 extends (the Y2 direction).

As illustrated in Figure 1, the axis lines La and Lb of the first roller 20a and the second roller 20b incline toward a side in the first direction Y1 of the longitudinal direction Y. As illustrated in Figure 2, an angle α1 formed by the axis line Lb of the second roller 20b viewed in the width direction X and an imaginary perpendicular R perpendicular to an imaginary plane S in contact with the four rollers 20a to 20d (i.e., a lateral projection angle) can be set to 0 to 40° and is set to 15° in the present embodiment. An angle formed by the axis line La of the first roller 20a and the imaginary perpendicular R (a lateral projection angle) is set as with α1.

The axis lines Lc and Ld of the third roller 20c and the fourth roller 20d incline toward a side in the second direction Y2 of the longitudinal direction Y. As illustrated in Figure 2, an angle α2 formed by the axis line Lc of the third roller 20c viewed in the width direction X and the imaginary perpendicular R of the imaginary plane S (i.e., an lateral projection angle) can be set to 20 to 65° and is set to 30° in the present embodiment. An angle formed by the axis line Ld of the fourth roller 20d and the imaginary perpendicular R (a lateral projection angle) is set as with α2.

That is, when viewed in the direction (the width direction X) of arranging the first pair of rollers (the first roller 20a and the second roller 20b), the first inclination angle α1 of the axis lines La and Lb of the first pair of rollers with respect to the imaginary perpendicular R is smaller than the second inclination angle α2 of the axis lines Lc and Ld of the second pair of rollers with respect to the imaginary perpendicular R.

As illustrated in Figure 1, a pair of the support shafts 40a and 40b supporting the first pair of rollers (the first roller 20a and the second roller 20b) extend spreading in the direction of arranging the first pair of rollers (the width direction X). That is, the interval between the support shaft 40a and the support shaft 40b in the width direction X (the direction of arranging the first pair of rollers) increases as the support shaft 40a and the support shaft 40b extend toward the front ends (the end portions on sides opposite to the main body 10). Similarly, a pair of the support shafts 40c and 40d supporting the second pair of rollers (the third roller 20c and the fourth roller 20d) extend spreading in the direction of arranging the second pair of rollers (the width direction X). That is, the interval between the support shaft 40c and the support shaft 40d in the width direction X (the direction of arranging the second pair of rollers) increases as the support shaft 40c and the support shaft 40d extend toward the front ends (the end portions on sides opposite to the main body 10).

As illustrated in Figure 5, an angle β1 formed by the axis line La of the first roller 20a and the axis line Lb of the second roller 20b can be set to 60 to 80°, preferably to 65 to 75°, and is set to 70° in the present embodiment. It is noted that the angle β1 formed by the axis line La and the axis line Lb is assumed to be an angle formed by both when viewed in the normal direction of a plane including the axis lines La and Lb.

As illustrated in Figure 6, an angle β2 formed by the axis line Lc of the third roller 20c and the axis line Ld of the fourth roller 20d can be set to 60 to 80°, preferably to 65 to 75°, and is set to 70° in the present embodiment. It is noted that the angle β2 formed by the axis line Lc and the axis line Ld is assumed to be an angle formed by both when viewed in the normal direction of a plane including the axis lines Lc and Ld.

As illustrated in Figure 3, a diameter D of the four rollers 20a to 20d can be set to 20 to 40 mm, preferably to 25 to 35 mm, and is set to 30 mm in the present embodiment. A distance W1 between the outer circumferential surface 21a of the first roller 20a and the outer circumferential surface 21b of the second roller 20b can be set to 8 to 12 mm, preferably to 8.5 to 10 mm, and is set to 9 mm in the present embodiment. A distance between the outer circumferential surface 21c of the third roller 20c and the outer circumferential surface 21d of the fourth roller 20d is equal to W1.

A distance W2 between the outer circumferential surface 21b of the second roller 20b and the outer circumferential surface 21c of the third roller 20c can be set to 12 mm to 20 mm, preferably to 14 to 18 mm, and is set to 15 mm in the present embodiment. A distance between the outer circumferential surface 21a of the first roller 20a and the outer circumferential surface 21d of the fourth roller 20d is equal to W2.

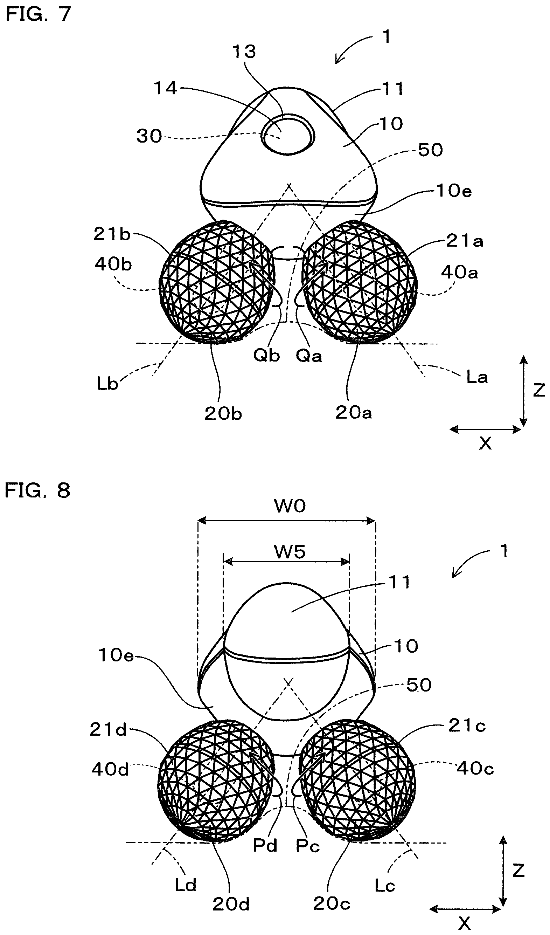

As illustrated in Figure 8, when viewed in a direction from the handle 11 to the main body, the supporting shaft mounting surface 10e is formed being wider than the handle 11. That is, the width of the supporting shaft mounting surface 10e (i.e., the length W0 of the main body 10 in the width direction X) is larger than a width W5 of the handle 11. In addition, as illustrated in Figure 2 to Figure 4, the rollers 20 are positioned on a side toward which the support shafts 40a to 40d extend from the supporting shaft mounting surface 10e.

As illustrated in Figure 1, in a central portion, on the front side, of the main body 10, the window portion 13 is formed. In the window portion 13, a lens 14 is fitted. In addition, on the back side of the lens 14 (on the inside of the main body 10), the photovoltaic panel 30 is arranged. The photovoltaic panel 30 can receive light through the lens 14.

The photovoltaic panel 30 is electrically connected to the conductive-platinum-plated four rollers 20a to 20d and the conductive-chromium-plated main body 10, via a terminal and the like (not illustrated). With this configuration, in use of the cosmetic device 1, the outer circumferential surfaces 21a to 21d of the rollers 20a to 20d are brought into contact with skin, and the rollers 20 and the main body 10 are electrically connected via a human body. Then, the photovoltaic panel 30 receives light through the lens 14 of the window portion 13 to generate electricity, causing weak current to flow between the outer circumferential surfaces 21a to 21d of the rollers 20a to 20d and the skin. As a result, stimuli to the skin are increased, further enhancing a massaging effect.

Now, usage of cosmetic device 1 will be described.

First, a user holds the cosmetic device 1 by the handle 11 and, in this state, moves the cosmetic device 1 in the first direction Y1 of the longitudinal direction Y (the direction of an arrow P) with the outer circumferential surfaces 21a to 21d of the four rollers 20a to 20d pressed against and brought into contact with a skin surface 50 (see Figure 2) of a portion like a face that curves in various directions. This movement causes, as illustrated in Figure 3, the first roller 20a to rotate in the direction of an arrow Pa and the second roller 20b to rotate in the direction of an arrow Pb. Then, the first roller 20a and the second roller 20b press the skin surface 50 (see Figure 2). Furthermore, as illustrated in Figure 8, the third roller 20c is caused to rotate in the direction of an arrow Pc, and the fourth roller 20d is caused to rotate in the direction of an arrow Pd. With this configuration, the third roller 20c and the fourth roller 20d pick up the skin surface 50 positioned between the third roller 20c and the fourth roller 20d as if to roll up the skin surface 50 as both the rollers rotate.

When the cosmetic device 1 is then moved in the second direction Y2 of the longitudinal direction Y in Figure 2 and Figure 3 (the direction of an arrow Q) so as to return the cosmetic device 1 to the original state, the first roller 20a is caused to rotate in the direction of an arrow Qa, and the second roller 20b is caused to rotate in the direction of an arrow Qb. With this configuration, as illustrated in Figure 7, the first roller 20a and the second roller 20d pick up the skin surface 50 positioned between the first roller 20a and the second roller 20b as if to roll up the skin surface 50 as both the rollers rotate. Furthermore, as illustrated in Figure 3, the third roller 20c is caused to rotate in the direction of an arrow Qc, the fourth roller 20d is caused to rotate in the direction of an arrow Qd, and the third roller 20c and the fourth roller 20d press the skin surface 50 (see Figure 2).

As illustrated in Figure 2, a user holds the handle 11 and, in this state, moves the cosmetic device 1 in the first direction X1 of the width direction X (a U direction) with the outer circumferential surfaces 21a to 21d of the four rollers 20a to 20d pressed against and brought into contact with a skin surface 50 of a portion like a face that curves in various directions. This movement causes, as illustrated in Figure 3, the first roller 20a to rotate in the direction of the arrow Pa, and the fourth roller 20c to rotate in the direction of the arrow Qd, whereby the skin surface 50 is pressed. Moreover, the second roller 20b is caused to rotate in the direction of the arrow Pb, and the third roller 20c is caused to rotate in the direction of the arrow Qc. Although not illustrated, with this configuration, the second roller 20b and the third roller 20c pick up the skin surface 50 positioned between the second roller 20b and the third roller 20c as if to roll up the skin surface 50 as both the rollers rotate.

When the cosmetic device 1 is then moved in the second direction X2 of the width direction X (a V direction) so as to return the cosmetic device 1 to the original state, the first roller 20a is caused to rotate in the direction of the arrow Qa, and the fourth roller 20c is caused to rotate in the direction of the arrow Pd, whereby the skin surface 50 is pressed. Moreover, the second roller 20b is caused to rotate in the direction of the arrow Qb, and the third roller 20c is caused to rotate in the direction of the arrow Pc. Although not illustrated, with this configuration, the first roller 20a and the fourth roller 20d pick up the skin surface 50 positioned between the first roller 20a and the fourth roller 20d as if to roll up the skin surface 50 as both the rollers rotate.

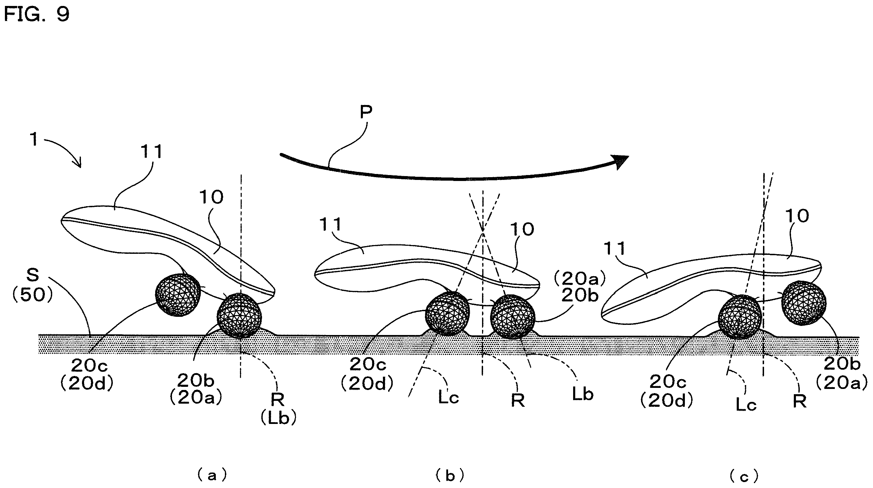

Moreover, the cosmetic device 1 can be used as follows. First, as illustrated in Figure 9(a), the cosmetic device 1 is moved in a direction from the second pair of rollers (the third roller 20c and the fourth roller 20d) toward the first pair of rollers (the first roller 20a and the second roller 20b) (a moving direction P), with the second pair of rollers (the third roller 20c and the fourth roller 20d) separated from a skin surface, and with the first pair of rollers (the first roller 20a and the second roller 20b) made to abut the skin surface, and then, as illustrated in Figure 9(b), the second pair of rollers (the third roller 20c and the fourth roller 20d) are made to abut the skin surface (the first stage). Subsequently, the cosmetic device 1 is moved in the moving direction P, with the first roller 20a, the second roller 20b, the third roller 20c, and the fourth roller 20d made to abut the skin surface 50 (the second stage). Furthermore, while the cosmetic device 1 is moved in the moving direction P, as illustrated in Figure 9C, the first pair of rollers (the first roller 20a and the second roller 20b) are made separated from the skin surface 50 (the third stage).

Through the above-described usage of the cosmetic device 1 in the present embodiment, first, the first stage has an effect of moderately picking up the skin surface 50 by the first pair of rollers (the first roller 20a and the second roller 20b) abutting the skin surface 50. Moreover, the second stage has an effect of keeping the skin picked up by the leading first pair of rollers (the first roller 20a and the second roller 20b), and moderately picking up the skin surface 50 by the following second pair of rollers (the third roller 20c and the fourth roller 20d). Subsequently, the third stage has an effect of releasing the picking up of the skin by the first pair of rollers (the first roller 20a and the second roller 20b), and continuing the moderately picking up of the skin surface 50 by the follow second pair of rollers (the third roller 20c and the fourth roller 20d).

In the present embodiment, as illustrated in Figure 2, in the state where the rollers are brought into contact with the skin surface 50, the angle α1 is smaller than the angle α2 when viewed in the direction of arranging the first pair of rollers (the first roller 20a and the second roller 20b), and thus, the amount of picking up the skin by the first pair of rollers (the first roller 20a and the second roller 20b) is larger than the amount of picking up the skin by the second pair of rollers (the third roller 20c and the fourth roller 20d). Therefore, the picking-up effect by the first pair of rollers (the first roller 20a and the second roller 20b) in the first stage and the second stage is exerted relatively weakly, and the picking-up effect by the second pair of rollers (the third roller 20c and the fourth roller 20d) in the second stage and the third stage is exerted relatively strongly. This configuration imparts differences in the skin-picking-up effect, further enhancing the massaging effect. In addition, exerting the picking-up effect in two strengths enables a user to recognize more clearly that skin is picked up, whereby the usability of the cosmetic device 1 can be enhanced.

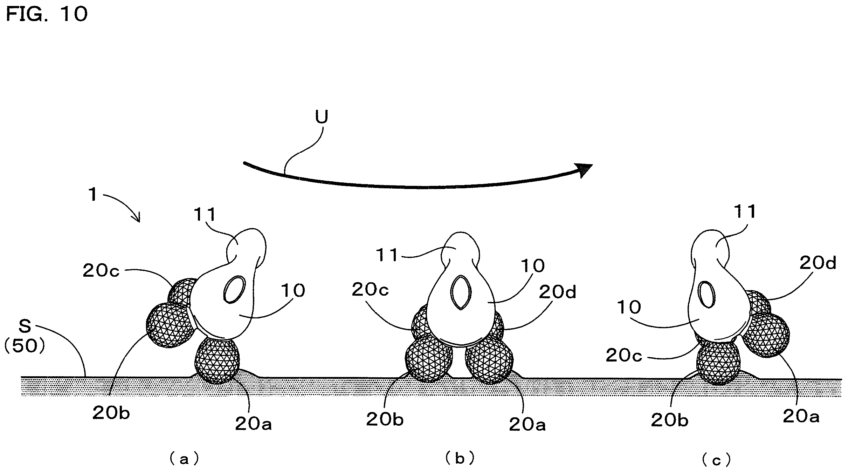

Moreover, the cosmetic device 1 can be used as follows. First, as illustrated in Figure 10(a), the cosmetic device 1 is moved in a direction from the second roller 20b toward the first roller 20a (a moving direction U), with the second roller 20b and the third roller 20c separated from a skin surface 50, and with the first roller 20a and the fourth roller 20d made to abut the skin surface 50, and then, as illustrated in Figure 10(b), the second roller 20b and the third roller 20c are made to abut the skin surface 50 (a fourth stage). Subsequently, the cosmetic device 1 is moved in the moving direction U, with the first roller 20a, the second roller 20b, the third roller 20c, and the fourth roller 20d made to abut the skin surface 50 (a fifth stage). Furthermore, while the cosmetic device 1 is moved in the moving direction U, as illustrated in Figure 10(c), the first roller 20a and the fourth roller 20d are made separated from the skin surface 50 (a sixth stage).

According to this usage, the fourth stage has an effect of moderately picking up the skin surface 50 by the leading first roller 20a and fourth roller 20d. Subsequently, the fifth stage has an effect of keeping the skin surface 50 picked up by the leading first roller 20a and fourth roller 20d, and moderately picking up the skin surface 50 by the following second roller 20b and third roller 20c. Moreover, the sixth stage has an effect of releasing the picking up of skin surface 50 by the first roller 20a and the fourth roller 20d, and continuing the moderately picking up of the skin surface 50 by the following second roller 20b and third roller 20c.

Now, operational advantages of the cosmetic device 1 in the present embodiment will be described.

In the cosmetic device 1 in the present embodiment, the main body 10 is provided with the handle 11. This configuration allows the cosmetic device 1 to be used with the handle 11 held, eliminating the necessity of excessively greatly changing the direction of a hand holding the handle 11 or the necessity of excessively greatly twisting a wrist when moving the rollers along skin of a portion like a face that curves in various directions. As a result, the user-friendliness of the cosmetic device 1 is improved as compared with the case where the main body 10 is directly held, and it is possible to facilitate the movement of the rollers 20 while keeping a proper angle with respect to a portion like a face that curves in various directions, providing a sufficient massaging effect.

In the present embodiment, the connecting portion 12 between the main body 10 and the handle 11 is narrowed and thus guides fingers along the narrowed portion of the connecting portion 12 when the handle 11 is held, making the handle 11 easy to hold. Thus, when the cosmetic device 1 is used for a portion like a face that curves in various directions, it is possible to hold the handle 11 sufficiently even when the direction of a hand holding the handle 11 is changed, whereby the usability of the cosmetic device 1 is enhanced.

In the present embodiment, as illustrated in Figure 2, the connecting portion 12 includes the surface 12a on a side (Z2 side) opposite to the side toward which the support shafts 40a to 40b extend so as to go away from each other, and a surface 12b on the side (Z1 side) toward which the support shafts 40a to 40b extends so as to go away from each other, and when viewed in a direction (the width direction X, see Figure 3) of arranging the first pair of rollers (the first roller 20a and the second roller 20b), the surface 12a is formed so as to have a gentle curve, and the surface 12b is formed so as to be recessed and narrowed. That is, the connecting portion 12 is narrowed so as to be recessed toward a side (in the Z2 direction) opposite to the side toward which the support shafts 40a to 40b extend. This configuration facilitates guiding the rollers 20 to a position on a palm side when the handle 11 is held, and thus facilitates keeping a proper angle of the rollers 20 with respect to skin. The user-friendliness of the cosmetic device 1 is thereby improved.

According to the cosmetic device 1 in the present embodiment, when viewed in the direction (the width direction X) of arranging the first pair of rollers (the first roller 20a and the second roller 20b), the first inclination angle α1 of the axis lines La and Lb of the first pair of rollers with respect to the imaginary perpendicular R is smaller than the second inclination angle α2 of the axis lines Lc and Ld of the second pair of rollers (the third roller 20c and the fourth roller 20d) with respect to the imaginary perpendicular R. This configuration has an effect of moderately picking up skin by the following pair of rollers also on the skin surface 50 of a curved portion. Moreover, the amount of picking up skin by the third roller 20c and the fourth roller 20d is larger than the amount of picking up skin by the first roller 20a and the second roller 20b. This configuration imparts differences in the skin-picking-up effect, further enhancing the massaging effect. In addition, exerting the picking-up effect in two strengths enables a user to recognize more clearly that skin is picked up, whereby the usability of the cosmetic device 1 can be enhanced.

In the present embodiment, the angle α1 formed by the axis line La (Lb) and the imaginary perpendicular R (lateral projection angle), the angle α2 formed by the axis line Lc (Ld) and the imaginary perpendicular R (lateral projection angle), the angle β1 formed by the axis line La and the axis line Lb, and the angle β2 formed by the axis line Lc and the axis line Ld are set as previously described. This configuration allows, of the four rollers 20a to 20d, the skin-picking-up effect by the following pair of rollers and the pressing effect by the leading pair of rollers to be exerted not excessively strongly or weakly but moderately on a portion like a face that curves in various directions. Therefore, the cosmetic device 1 exerts the massaging effect effectively on these portions.

In the present embodiment, all the four rollers 20a to 20d are formed to be spherical, and the axis lines La to Ld of the rollers 20a to 20d incline with respect to one another. With this configuration, when the rollers 20a to 20d are subjected to reciprocating operation while being brought into contact with the skin surface 50, the skin-picking-up effect can be exerted effectively by the following pair of rollers of the rollers 20a to 20d adjacent to one another, without pressing the rollers 20a to 20d against skin.

As to the shape of the four rollers 20a to 20d, an oval-sphere shape, or a shape of the combination of oval-sphere shapes may be employed. On the outer circumferential surfaces 21a to 21d of the rollers 20a to 20d, unevenness or protrusions of various shapes may be provided. The outer circumferential surfaces 21a to 21d may be formed to be smooth surfaces, or granular surfaces. Each of the rollers 20a to 20d may be made up of a single member or made up by combining two or more members.

In the present embodiment, the handle 11 extends from the main body 10 in the direction from the first pair of rollers (the first roller 20a and the second roller 20b) toward the second pair of rollers (the third roller 20c and the fourth roller 20d). This configuration facilitates a reciprocating operation of the cosmetic device 1 in the direction Y from the first pair of rollers toward the second pair of rollers, with the handle 11 held. As a result, it is possible to exert easily, of the first pair of rollers and the second pair of rollers, the skin-pressing effect by the leading pair of rollers and the picking-up effect by the following pair of rollers, whereby the operability of the cosmetic device 1 is enhanced.

Since the four support shafts 40a to 40d extend so as to go away from each other, the movement of the cosmetic device 1 in one direction causes both skin pressing by the leading pair of rollers and skin picking up by the following pair of rollers. Moreover, in addition to in one direction, the reciprocating motion of the cosmetic device 1 in a plurality of directions (a PQ direction and a UV direction in the present embodiment) also exerts the massaging effect. The operability of the cosmetic device 1 is thereby enhanced when the cosmetic device 1 is used for a portion like a face that curves in various directions.

Since the handle 11 extends from the main body 10 in a direction different from the directions of the support shafts 40a to 40d, interference between a hand and the rollers 20a to 20d is restricted when the handle 11 is held, whereby the handle 11 is made easy to hold. As a result, the cosmetic device 1 is made easy to move with the rollers 20a to 20d running along a portion like a face that curves in various directions, whereby the operability of the cosmetic device 1 is enhanced.

Although the handle 11 is assumed in the present embodiment to extend linearly in the direction from the first pair of rollers toward the second pair of rollers but is not limited to this configuration. The handle 11 may extend in a direction from the first pair of rollers toward the second pair of rollers and curve in the thickness direction Z or the width direction X. In addition, although the handle 11 is assumed in the present embodiment to extend from the end portion, in the longitudinal direction Y, of the main body 10 but is not limited to this configuration. For example, the handle 11 may extend from a substantially middle of the main body 10. In addition, the handle 11 may be made up of a member other than the member of the main body 10 and attached to the main body 10 so as to extend from the main body 10. The number of handles 11 is not limited to one, and a plurality of handles may be provided. In the case of providing a plurality of handles, the plurality of handles may extend in directions different from one another.

The handle 11 has a substantially stick shape in plan view, including the swelling central portion 11c and the narrowed connecting portion 12 that is connected to the main body 10. This configuration makes the handle 11 easy to hold and prevents a hand holding the handle 11 from interfering with the rollers 20a to 20d provided in the main body 10, whereby the operability of the cosmetic device 1 is further enhanced. In addition, in the main body 10, when viewed laterally as illustrated in Figure 2, the connecting portion 12 between the handle 11 and the main body 10 is narrowed, and the handle 11 slightly inclines toward a body base 10a side with respect to the main body 10. This configuration makes it easy to bring the rollers 20a to 20d into contact with the skin surface 50 of a portion like a face that curves in various directions with the handle 11 held, making the cosmetic device 1 excellent in operability.

When viewed from a side toward which the support shafts 40a to 40d extend, the main body 10 is formed so as to be wider than the connecting portion 12. This configuration increases the rigidity of the main body 10, inhibiting the main body from yielding at a load applied to the main body 10 from the rollers 20 or the handle 11 in use. It is thereby possible to cause the rollers 20 to abut the skin surface 50 reliably, whereby providing a high massaging effect even in massaging a curved portion. In addition, the main body 10 is therefore excellent in durability, whereby the cosmetic device 1 is made suitable to use for a portion that curves in various directions.

The support shafts 40a to 40d are covered with the main body 10 and the rollers 20 and are not exposed. This configuration prevents skin, hair, or the like from contacting with the support shafts 40a to 40d in use. It is thus possible to prevent a user from bearing an unnecessary burden, whereby the usability of the cosmetic device 1 is enhanced.

In the present embodiment, the diameter D of the four rollers 20a to 20d, the distance W1 between the outer circumferential surfaces 21a and 21b (between 21c and 21d), and the distance W2 between the outer circumferential surfaces 21b and 21c (between 21a and 21d) are set as previously described. This configuration makes the size of a zone for contacting with the four rollers 20a to 20d not excessively large or small but appropriate in use for a portion like a face that curves in various directions, and thus the cosmetic device 1 is suitable to use for such a portion. In addition, the rollers 20a to 20d are made to have a moderate size and thus well-balanced in weight, whereby the cosmetic device 1 is made excellent in operability.

In the present embodiment, as illustrated in Figure 8, when viewed in a direction from the handle 11 to the main body, the supporting shaft mounting surface 10e, which is a surface on a side on which the support shafts 40a to 40d are provided in the main body 10, is formed so as to be wider than the handle 11, the support shafts 40a to 40d are provided through the supporting shaft mounting surface 10e at the predetermined intervals, and the rollers 20 are positioned on a side toward which the support shafts 40a to 40d extend from the supporting shaft mounting surface 10e. This configuration enables the adjacent rollers 20 to be disposed at the predetermined intervals while maintaining the rigidity of the main body 10. It is thereby possible to retain the adjacent rollers 20 at the predetermined intervals even when the handle 11 is held to press the rollers 20 against the skin surface of a portion like a face that curves in various directions. This retention enables moderate skin-pressing effect and the skin-picking-up effect to be exerted more effectively on a curved portion, whereby the cosmetic device 1 is made suitable to use for a portion that curves in various directions.

In the present embodiment, the handle 11 is substantially rigid. This configuration makes the handle 11 hard to yield, whereby force given to the handle 11 can be exactly transmitted to the rollers 20. Therefore, by changing the strength of force given to the handle 11, it is possible to perform pressing and picking up with an appropriate strength for a portion to massage, exerting a high massaging effect.

Although there are provided the four support shafts 40a to 40d in the present embodiment, provision of at least four support shafts suffices, and five or more support shafts may be provided. Provision of five or more support shafts can also bring the same operational advantages as in the present embodiment.

In the present embodiment, all the four support shafts 40a to 40d are fixed to the main body 10 at the first end portion 42a to 42d, and rotatably support the rollers 20a to 20d at the second end portions 43a to 43d. Instead of this configuration, part or all of the four support shafts 40a to 40d may have the second end portions 43a to 43d fixed to the rollers 20a to 20d and may be rotatably attached to the main body 10 at the first end portion 42a to 42d, whereby the support shafts 40a to 40d support the rollers 20a to 20d in such a manner that the support shafts 40a to 40d are rotatable integrally with the rollers 20a to 20d. In this case, the main body 10 may be provided with bearings or the like that make the support shafts 40a to 40d rotatable, eliminating the necessity of providing a structure inside the rollers 20a to 20d to make the rollers 20a to 20d rotatable, whereby the freedom in design of the rollers 20a to 20d is enhanced. The configuration of this case can also bring the same operational advantages as in the present embodiment.

As seen from the above, according to the present embodiment, it is possible to provide the cosmetic device 1 that is suitable to massage a portion like a face that curves in various directions.

### (EXAMPLE)

Next, an evaluation was made on the usability of the cosmetic device 1 when the angle α1 formed by the axis line La (Lb) and the imaginary perpendicular R (lateral projection angle), the angle α2 formed by the axis line Lc (Ld) and the imaginary perpendicular R (lateral projection angle) were changed.

The evaluation test was conducted by a method in which cosmetic devices 1 were used in a cheek of the face of an examinee, and the cosmetic devices 1 had lateral projection angles α1 that were changed from 0 to 70° in 5° intervals, and lateral projection angles α2 that were changed from 0 to 80° in 5° intervals. The number of examinees was one, a Japanese adult male.

The evaluation was made by rating the usability with four grades (Very good: ⊚, Good: ○, Not very good: Δ, Bad: ×). The results of the evaluation are shown in Table 1.
[Table 1]

**(Table 1)**

| α1(°) | Evaluation | α2(°) | Evaluation |
|---|---|---|---|
| 0 | ○ | 0 | Δ |
| 5 | ○ | 5 | Δ |
| 10 | ○ | 10 | Δ |
| 15 | ⊚ | 15 | Δ |
| 20 | ○ | 20 | ○ |
| 25 | ○ | 25 | ○ |
| 30 | ○ | 30 | ⊚ |
| 35 | ○ | 35 | ○ |
| 40 | ○ | 40 | ○ |
| 45 | Δ | 45 | ○ |
| 50 | Δ | 50 | ○ |
| 55 | Δ | 55 | ○ |
| 60 | Δ | 60 | ○ |
| 65 | Δ | 65 | ○ |
| 70 | × | 70 | Δ |
| - | - | 75 | Δ |
| - | - | 80 | × |

As shown in Table 1, it was found that the lateral projection angle α1 is the most preferable at 15° and preferable from 0 to 40°. It was also found that the lateral projection angle α2 is the most preferable at 30° and preferable from 20 to 65°.

## Claims

1. A cosmetic device comprising:
a main body;
four support shafts that are supported by the main body and extend from the main body so as to go away from each other;
rollers that are supported by the support shafts so as to be rotatable about axis lines of the support shafts; and
a handle that extends from the main body.

2. The cosmetic device according to claim 1, wherein the rollers include a first pair of rollers that are adjacent to each other, and a second pair of rollers that are adjacent to each other, and when viewed in a direction arranging the first pair of rollers, a first inclination angle of axis lines of the first pair of rollers with respect to an imaginary perpendicular is smaller than a second inclination angle of axis lines of the second pair of rollers with respect to the imaginary perpendicular, the imaginary perpendicular being perpendicular to an imaginary plane in contact with the rollers.

3. The cosmetic device according to claim 2, wherein the handle extends in a direction from the first pair of rollers toward the second pair of rollers.

4. The cosmetic device according to any one of claims 1 to 3, wherein the support shafts are covered with the main body and the rollers and are not exposed.

5. The cosmetic device according to any one of claims 1 to 4, wherein, when viewed in a direction from the handle to the main body, a supporting shaft mounting surface, which is a surface on a side on which the support shafts are provided in the main body, is formed so as to be wider than the handle, the support shafts are provided through the supporting shaft mounting surface at predetermined intervals, and the rollers are positioned on a side toward which the support shafts extend from the supporting shaft mounting surface.

6. The cosmetic device according to any one of claims 1 to 5, wherein the handle is substantially rigid.

7. The cosmetic device according to any one of claims 1 to 6, wherein the support shafts are rotatably supported by the main body, and the rollers are supported by the support shafts in such a manner that the rollers are rotatable integrally with the support shafts.
